# EUROPEAN PATENT APPLICATION

(11) **EP 2 316 435 A1**
(43) Date of publication of application: **04.05.2011**
(21) Application number: 09173724.7
(22) Date of filing: 22.10.2009
(51) Int. Cl.: A61K 9/24, A61K 31/4985, A61K 31/138

(54) **Pharmaceutical compositions of PDE-5 inhibitors and dapoxetine**

(71) Applicant: Abdi Ibrahim Ilac Sanayi Ve Ticaret Anonim Sirketi, 34555 Hadimkoy, Istanbul (TR)
(72) Inventor: Farshi, Farhad, 34555 ISTANBUL (TR); Koc, Fikret, 34555 ISTANBUL (TR); Soylemez, Serdar, 34555 ISTANBUL (TR); Kandemir, Levent, 34555 ISTANBUL (TR)

(57) **Abstract**

This invention is related to pharmaceutical formulations of selective type 5 PDE-5 inhibitors and selective serotonin reuptake inhibitor (SSRI) which is used in the treatment of premature ejaculation, such as dapoxetine, combination. This invention is also directed to preparation methods for said combination.
The preferred combination is tadalafil with dapoxetine. The pharmaceutical formulations are in form of a tablet-in-tablet, a bilayer tablet or kit.

## Description

### Technical Field

This invention is related to pharmaceutical formulations of selective type 5 PDE-5 inhibitors and selective serotonin reuptake inhibitor (SSRI) which is used in the treatment of premature ejaculation, such as dapoxetine, combination. This invention is also directed to preparation methods for said combination.

### Background Art

PCT/WO 03000343 (VIVUS, INC.) 21.06.2001 discloses phosphodiesterase inhibitor and dapoxetine combination is administered transmucosally, e.g., via the sublingual, buccal, nasal, or rectal routes, or via inhalation.

Dapoxetine and PDE-5 inhibitors combination is known in the prior art. For example ; DRESSER, et al. "Dapoxetine, a novel treatment for premature ejaculation, does not have pharmacokinetic interactions with phosphodiesterase-5 inhibitors". Int. J.Import Res.. 2006, vol.18, no.1.

### Disclosure of Invention

Selective type 5 cGMP phosphodiesterase (PDE-5) inhibitors are used in treatment of erectile dysfunction and have a elimination half life of at least 4-5 hours in the case of tadalafil the elimination half life is about 17 hours.

Dapoxetine hydrochloride (Priligy®) is a short-acting SSRI drug for the treatment of premature ejaculation in men and has an elimination half-life of approximatley 1.5 h.

This invention is related to pharmaceutical compositions of a PDE-5 inhibitor or mixtures of PDE-5 inhibitors or pharmaceutically acceptable salts thereof and a selective serotonin reuptake inhibitor (SSRI) or mixtures of selective serotonin reuptake inhibitors or pharmaceutically acceptable salts thereof wherein the selective serotonin reuptake inhibitors are used in the treatment of premature ejaculation.

Another issue of this invention is related to close the big difference in elimination half life between dapoxetine and PDE-5 inhibitors by using different sustained or modified release but not limited to these formulations.

These formulations aim for modification of the release profile to have efficient dapoxetine blood level concentration for a longer period of time than without modification.

According to this invention the term "phosphodiesterase inhibitor" or "PDE 5 inhibitor" includes phosphodiesterase inhibitors per se as well as pharmaceutically acceptable and pharmacologically active salts, esters, amides, prodrugs, active metabolites, other derivatives, mixtures thereof and the like .

The term "therapeutically effective amount" is meant a nontoxic but sufficient amount of the drug or agent to provide the desired effect.

According to this invention the term "selective serotonin reuptake inhibitor" (SSRI) includes selective serotonin reuptake inhibitors per se as well as pharmaceutically acceptable and pharmacologically active salts, esters, amides, prodrugs, active metabolites, other derivatives, mixtures thereof and the like .

PDE5 selective inhibitors are, but not limited to, sildenafil, tadalafil, vardenafil, udenafil, avanafil, dasantafil, SLx2101 and LAS34179, mixtures thereof and the like. Preferred PDE5 inhibitor is tadalafil.

Serotonin reuptake inhibitors (SSRI), which used in the treatment of premature ejaculation, are but not limited to fluoxetine, dapoxetine, sertraline, citalopram, escitalopram,venlafaxine, mixtrures thereof and the like. Preferred serotonin reuptake inhibitor is dapoxetine and especially its hydrochloride salt.

According to this invention pharmaceutical compositions may be in the form of tablet, bilayer tablet, tablet in tablet, capsule, tablet in capsule, pellets in capsule, granules in capsule and other dosage forms suitable for oral administration.

In one embodiment, the present invention provides tablet-in-tablet compositions comprising: a) a core tablet comprising: dapoxetine or pharmaceutically acceptable salts thereof and a pharmaceutically acceptable carrier or carriers b) a compressed outer tablet layer comprising: a PDE 5 inhibitor or mixtures of PDE 5 inhibitors or pharmaceutically acceptable salts thereof and a pharmaceutically acceptable carrier or carriers

In another embodiment, this invention embraces bilayer pharmaceutical tablet. Bilayer pharmaceutical tablet is comprising; a) a first layer containing a PDE 5 inhibitor or mixtures of PDE 5 inhibitors or pharmaceutically acceptable salts thereof and a pharmaceutically acceptable carrier or carriers and b) a second layer containing dapoxetine or pharmaceutically acceptable salts thereof and a pharmaceutically acceptable carrier or carriers

Pharmaceutical formulations of combination can be prepared by one of the method as following manners; a) Active pharmaceutical ingredients are granulated seperately and then granulates are assemblied namely the active pharmaceutical ingredients are each dispersed within its own pharmaceutically acceptable carrier or carriers. One of them may be direct compression (DC) form. b) One active pharmaceutical ingredient is granulated and then granulated active pharmaceutical ingredient is assemblied with non granulated active pharmaceutical ingredient. c) Both active pharmaceutical ingredients are granulated together. d) One active pharmaceutical ingredient is dissolved alone or with one or more excipient and then other active pharmaceutical ingredient alone or with one or more excipients are coated with said solution. e) One active pharmaceutical ingredient is dispersed alone or with one or more excipients and then other active pharmaceutical ingredient alone or with one or more excipients are coated with said dispersion.

In the course of the preparation, direct compression, wet granulation, dry granulation methods or mixtures thereof can be applied.

Prepared granulates can be filled into capsules. Capsules can be consisted of separated compartments and each active ingredient and it's granulates can be filled into each compartment.

Modified release formulations can be preferred. Modified release formulations are, but not limited to, controlled release, sustained release, delayed release, extended release, repeat action system, mixtures thereof and the like.

Pharmaceutical formulations can be made in two parts wherein one part is modified release formulation of PDE 5 inhibitor and another one is immediate release formulation of selective serotonin reuptake inhibitor (SSRI) or vice versa.

In one embodiment, the present invention provides tablet-in-tablet compositions whereby the core tablet has a modified release formulation comprising: a) a core tablet comprising: dapoxetine or pharmaceutically acceptable salts thereof and a pharmaceutically acceptable carrier or carriers to provide dapoxetine in a release profile to have efficient dapoxetine blood level concentration for a longer period of time than without modification when compared with separate formulation of dapoxetine which is launched under Priligy® trademark. b) a compressed outer tablet layer comprising: a PDE 5 inhibitor or mixtures of PDE 5 inhibitors or pharmaceutically acceptable salts thereof and a pharmaceutically acceptable carrier or carriers

In another embodiment, the present invention provides tablet-in-tablet compositions whereby the core tablet has a modified release formulation comprising: a) a core tablet comprising: an effective amount of dapoxetine or pharmaceutically acceptable salts thereof and a pharmaceutically acceptable carrier or carriers to provide dapoxetine in release profile to have efficient dapoxetine blood level concentration for a longer period of time than without modification when compared with separate formulation of dapoxetine which is launched under Priligy® trademark. b) a compressed outer tablet layer comprising: a PDE 5 inhibitor or mixtures of PDE 5 inhibitors or pharmaceutically acceptable salts thereof and a initial amount of dapoxetine to provide an efficient amount of dapoxetine for initial effectively and a pharmaceutically acceptable carrier or carriers

In another embodiment, this invention embraces bilayer pharmaceutical tablet. Bilayer pharmaceutical tablet is comprising; a) a first layer containing a PDE 5 inhibitor or mixtures of PDE 5 inhibitors or pharmaceutically acceptable salts thereof and a pharmaceutically acceptable carrier or carriers and b) a second layer containing dapoxetine or pharmaceutically acceptable salts thereof and a pharmaceutically acceptable carrier or carriers to provide dapoxetine in a release profile to have efficient dapoxetine blood level concentration for a longer period of time than without modification

In another embodiment, this invention embraces bilayer pharmaceutical tablet. Bilayer pharmaceutical tablet is comprising; a) first layer containing an effective amount of dapoxetine or pharmaceutically acceptable salts thereof and a pharmaceutically acceptable carrier or carriers to provide dapoxetine in release profile to have efficient dapoxetine blood level concentration for a longer period of time than without modification when compared with separate formulation of dapoxetine which is launched under Priligy® trademark. b) a second layer containing a PDE 5 inhibitor or mixtures of PDE 5 inhibitors or pharmaceutically acceptable salts thereof and a initial amount of dapoxetine to provide an efficient amount of dapoxetine for initial effectively and a pharmaceutically acceptable carrier or carriers

The expression bilayer is not limited to two layers also multilayer tablets are meant with this expression.

PDE-5 inhibitors and selective serotonin reuptake inhibitor (SSRI) combination can be administered in therapeutically effective amounts.

According to this invention, combination includes one or more pharmaceutically acceptable excipients, carriers, or diluents. In formulations, surfactants, diluents, sweeteners, disintegrants, binders, lubricants, glidants, colorants, flavors and mixtures thereof can be used.

Diluents are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch , mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like .

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate, mixtures thereof or whatsoever.

This invention embraces pharmaceutical composition of therapeutically effective amount of PDE 5 inhibitor or mixtures of PDE 5 inhibitors or pharmaceutically acceptable salts thereof and therapeutically effective amount of selective serotonin reuptake inhibitor or mixtures of selective serotonin reuptake inhibitors or pharmaceutically acceptable salts thereof and use of said composition for the manufacture of a medicament for the treatment of disease or diseases where premature ejaculation is associated with erectile dysfunction.

Another aspect of this invention is relevant to kits comprising (i) therapeutically effective amount of PDE 5 inhibitor or mixtures of PDE 5 inhibitors or its pharmaceutically acceptable forms or/and derivatives and a pharmaceutically acceptable carrier or diluent in a unit dosage form and (ii) therapeutically effective amount of selective serotonin reuptake inhibitor or mixtures of selective serotonin reuptake inhibitors or a pharmaceutically acceptable forms or/and derivatives and pharmaceutically acceptable carrier or diluent in a unit dosage form and (iii) a container.

The kits of the invention include container as separately compositions such as a divided bottle or a divided packet. Separately unit dosage forms can also be contained undivided container. Unit dosage forms can each be discrete dosage forms that are packaged together. The pharmaceutical kits can include instructions as a separate sheet or optionally printed on the containers.

In the kits, unit dosage forms are generally administered in the form of a pharmaceutical composition and each unit dosage form additionally comprising a pharmaceutically acceptable carrier or diluent.

### Example 1

**Table 1**

| Ingredients | Weight (mg) |
|---|---|
| Tadalafil | 20 |
| Dapoxetine | 60 |
| Lactose Monohydrate | 160 |
| Lactose Monohydrate (spray dried) | 30 |
| Hydroxypropylcellulose | 6 |
| Sodium Lauryl Sulfate | 3 |
| Microcrystalline Cellulose | 45 |
| Magnesium Stearate | 3 |
| Colloidal Silicon Dioxide | 7 |
| Croscarmellose Sodium | 10 |

## Claims

1. A tablet-in-tablet composition wherein: a) a core tablet comprising: a PDE 5 inhibitor or mixtures of PDE 5 inhibitors or their pharmaceutically acceptable salts and a pharmaceutically acceptable carrier or carriers b) a compressed outer tablet layer comprising: a selective serotonin reuptake inhibitor or mixtures of selective serotonin reuptake inhibitors or their pharmaceutically acceptable salts and a pharmaceutically acceptable carrier or carriers

2. A bilayer tablet wherein; a) a first layer containing a PDE 5 inhibitor or mixtures of PDE 5 inhibitors or their pharmaceutically acceptable salts and a pharmaceutically acceptable carrier or carriers and b) a second layer containing a selective serotonin reuptake inhibitor or mixtures of selective serotonin reuptake inhibitors or their pharmaceutically acceptable salts and a pharmaceutically acceptable carrier or carriers

3. A tablet-in-tablet composition, **characterized in that** the core tablet has a modified release formulation, comprising: a) a core tablet comprising:
dapoxetine or pharmaceutically acceptable salts thereof and a pharmaceutically acceptable carrier or carriers to provide dapoxetine in a release profile to have efficient dapoxetine blood level concentration for a longer period of time than without modification when compared with separate formulation of dapoxetine which is launched under Priligy® trademark. b) a compressed outer tablet layer comprising: a PDE 5 inhibitor or mixtures of PDE 5 inhibitors or pharmaceutically acceptable salts thereof and a pharmaceutically acceptable carrier or carriers

4. A tablet-in-tablet composition, **characterized in that** the core tablet has a modified release formulation, comprising: a) a core tablet comprising: an effective amount of dapoxetine or pharmaceutically acceptable salts thereof and a pharmaceutically acceptable carrier or carriers to provide dapoxetine in release profile to have efficient dapoxetine blood level concentration for a longer period of time than without modification when compared with separate formulation of dapoxetine which is launched under Priligy® trademark. b) a compressed outer tablet layer comprising: a PDE 5 inhibitor or mixtures of PDE 5 inhibitors or pharmaceutically acceptable salts thereof and a initial amount of dapoxetine to provide an efficient amount of dapoxetine for initial effectively and a pharmaceutically acceptable carrier or carriers

5. A bilayer tablet comprising; a) a first layer containing a PDE 5 inhibitor or mixtures of PDE 5 inhibitors or pharmaceutically acceptable salts thereof and a pharmaceutically acceptable carrier or carriers and b) a second layer containing dapoxetine or pharmaceutically acceptable salts thereof and a pharmaceutically acceptable carrier or carriers to provide dapoxetine in a release profile to have efficient dapoxetine blood level concentration for a longer period of time than without modification

6. A bilayer tablet comprising; a) first layer containing an effective amount of dapoxetine or pharmaceutically acceptable salts thereof and a pharmaceutically acceptable carrier or carriers to provide dapoxetine in release profile to have efficient dapoxetine blood level concentration for a longer period of time than without modification when compared with separate formulation of dapoxetine which is launched under Priligy® trademark. b) a second layer containing a PDE 5 inhibitor or mixtures of PDE 5 inhibitors or pharmaceutically acceptable salts thereof and a initial amount of dapoxetine to provide an efficient amount of dapoxetine for initial effectively and a pharmaceutically acceptable carrier or carriers

7. A preparation method of a PDE 5 inhibitor or mixtures of PDE 5 inhibitors or their pharmaceutically acceptable salts and a selective serotonin reuptake inhibitor or mixtures of selective serotonin reuptake inhibitors or their pharmaceutically acceptable salts combination **characterized in that** both active pharmaceutical ingredients are separately granulated and then granulates are assemblied .

8. A preparation method of a PDE 5 inhibitor or mixtures of PDE 5 inhibitors or their pharmaceutically acceptable salts and a selective serotonin reuptake inhibitor or mixtures of selective serotonin reuptake inhibitors or their pharmaceutically acceptable salts combination **characterized in that** both active pharmaceutical ingredients are granulated together.

9. A preparation method of a PDE 5 inhibitor or mixtures of PDE 5 inhibitors or their pharmaceutically acceptable salts and a selective serotonin reuptake inhibitor or mixtures of selective serotonin reuptake inhibitors or their pharmaceutically acceptable salts combination **characterized in that** one of the active pharmaceutical ingredient is granulated and then granulated active pharmaceutical ingredient is assemblied with non granulated active pharmaceutical ingredient

10. A preparation method of a PDE 5 inhibitor or mixtures of PDE 5 inhibitors or their pharmaceutically acceptable salts and a selective serotonin reuptake inhibitor or mixtures of selective serotonin reuptake inhibitors or their pharmaceutically acceptable salts combination **characterized in that** one of the active pharmaceutical ingredient is dissolved alone or with one or more excipients and then the other active pharmaceutical ingredient alone or with one or more excipients are coated with said solution.

11. A preparation method of a PDE 5 inhibitor or mixtures of PDE 5 inhibitors or their pharmaceutically acceptable salts and a selective serotonin reuptake inhibitor or mixtures of selective serotonin reuptake inhibitors or their pharmaceutically acceptable salts combination **characterized in that** one of the active pharmaceutical ingredient is dispersed alone or with one or more excipients and then other active pharmaceutical ingredient alone or with one or more excipients are coated or adhered with said dispersion

12. A pharmaceutical composition of therapeutically effective amount of PDE 5 inhibitor or mixtures of PDE 5 inhibitors or their pharmaceutically acceptable salts and therapeutically effective amount of selective serotonin reuptake inhibitor or mixtures of selective serotonin reuptake inhibitors or their pharmaceutically acceptable salts and use of said composition for the manufacture of a medicament for the treatment of premature ejaculation where it is associated with erectile dysfunction.

13. A kit comprising (i) therapeutically effective amount of PDE 5 inhibitor or mixtures of PDE 5 inhibitors or their pharmaceutically acceptable salts and a pharmaceutically acceptable carrier or diluent in a unit dosage form and (ii) therapeutically effective amount of selective serotonin reuptake inhibitor or mixtures of selective serotonin reuptake inhibitors or their pharmaceutically acceptable salts and pharmaceutically acceptable carrier or diluent in a unit dosage form and (iii) a container.

14. According to preceding claims PDE 5 inhibitor is per se as well as pharmaceutically acceptable and pharmacologically active salts, esters, amides, pro-drugs, active metabolites, other derivatives or mixtures thereof.

15. According to claim 14 PDE5 inhibitors are selected from the group comprising of sildenafil, tadalafil, vardenafil, udenafil, avanafil, dasantafil, SLx21 01 and LAS34179 or mixtures thereof.

16. According to claim 15 PDE5 inhibitor is tadalafil

17. According to claims of 1 to 13 selective serotonin reuptake inhibitor is per se as well as pharmaceutically acceptable and pharmacologically active salts, esters, amides, pro-drugs, active metabolites, other derivatives or mixtures thereof.

18. According claim 17, serotonin reuptake inhibitors (SSRI), are selected from the group comprising of fluoxetine, dapoxetine, sertraline, citalopram, escitalopram,venlafaxine or mixtures thereof.

19. According to claim 18 serotonin reuptake inhibitor is dapoxetine

20. According to claim 19 dapoxetine is dapoxetine hydrochloride
